# FASCICULE DE BREVET EUROPEEN

(11) **EP 2 570 101 B1**
(45) Date de publication et mention de la délivrance du brevet: **18.03.2015**
(21) Numéro de dépôt: 12181744.9
(22) Date de dépôt: 24.08.2012
(51) Int. Cl.: A61F 2/16

(54) **Dispositif perfectionné d'haptiques pour implant de sulcus**
Perfektionierte Vorrichtung zum haptischen Handhaben für Sulkus-Implantat
Improved haptic device for sulcus implant

(30) Priorité: 14.09.2011 FR 1158196
(43) Date de publication de la demande: 20.03.2013
(73) Titulaire: QMP Holding GmbH, 68163 Mannheim (DE)
(72) Inventeur: Dworschak, Rüdiger, 67146 Deidesheim (DE); Kontur, László, 80638 München (DE)
(74) Mandataire: Petit, Maxime

(56) Documents cités:
- WO-A1-01/17461
- WO-A1-2008/036674
- FR-A1- 2 776 181
- FR-A1- 2 819 713
- US-A- 4 257 130
- US-A1- 2002 120 331
- US-A1- 2007 142 911

## Description

L'invention concerne les lentilles implantables dans l'oeil généralement appelées lentilles intraoculaires, ou IOL selon leur acronyme anglo-saxon le plus souvent utilisé. Elle est plus spécialement relative aux lentilles destinées à être implantées dans le sulcus ciliaire, entre l'iris et le sac capsulaire, aussi bien dans le cas où ce dernier, après ablation du cristallin, n'est pas apte à recevoir une lentille correctrice, que dans le cas où le dit sac capsulaire contient déjà une lentille intraoculaire, la lentille de sulcus étant alors destinée à corriger les déficiences résiduelles ou résultantes après l'implantation d'une lentille principale dans le sac capsulaire, ou encore que dans le cas où il est nécessaire d'ajouter des caractéristiques nouvelles au système optique de l'oeil telles que :
- une correction de l'astigmatisme cornéen,
- une modification de la vision de près au moyen de lentilles multifocales,
- la mise en place d'un filtre de la lumière bleue en cas de sensibilité à la lumière.

Le document FR 2 776 181 décrit une lentille intraoculaire comprenant une partie optique et une partie haptique. La partie haptique comprend deux anses disposées symétriquement de part et d'autre de la partie optique. Chacune des anses de la partie haptique comporte plus particulièrement deux segments inférieurs reliés à la partie optique par une zone de raccordement et un segment supérieur qui relie les segments inférieurs entre eux. Chacun des segments inférieurs présente deux coudes formant points de fléchissement qui sont disposés entre le segment supérieur et chacune des zones de raccordement de la partie optique à l'un des segments inférieurs.

L'inconvénient des lentilles de sulcus connues est qu'elles ont une tendance naturelle à tourner et se décentrer en raison de la structure anatomique très irrégulière du sulcus ciliaire et de l'instabilité de ses mouvements.

L'invention vise à remédier à cet inconvénient et à procurer des lentilles intraoculaires de sulcus dont les haptiques assurent un maintien parfait de la partie optique quelque soit la structure anatomique du sulcus de l'oeil du patient dans lequel on les implante, et qui ne présentent pas le risque de rotation et de décentrement des lentilles de sulcus connues.

Dans ce but, l'invention est constituée par une lentille intraoculaire destinée à être implantée dans le sulcus ciliaire de l'oeil et qui comporte une partie optique à la périphérie de laquelle sont disposées au moins deux anses haptiques en forme de boucles fermées disposées symétriquement de part et d'autre de la partie optique, chaque boucle étant formée par au moins trois segments, un segment supérieur arqué et deux segments inférieurs assurant la liaison entre le segment supérieur et la partie optique, lesdits segments inférieurs étant convergents en direction de la partie optique, **caractérisée en ce que** les segments de chaque boucle sont raccordés entre eux par des points de fléchissement, en ce que les deux segments inférieurs se raccordent à la partie optique de la lentille par des points de fléchissement, et en ce que la longueur du segment supérieur est inférieure à la somme des longueurs des segments inférieurs et de leur écartement au niveau de leur raccordement à la partie optique.

Ainsi, les haptiques, sous les forces de compression exercées par les structures de l'oeil en direction de la partie optique, ne peuvent pas se déformer au-delà du point d'extension maximale dans lequel les deux segments supérieurs se trouvent en alignement l'un avec l'autre. Ceci procure une grande flexibilité de la partie haute des haptiques en contact avec le sulcus ciliaire tout en limitant la déformation à un diamètre cible minimal, de préférence compris entre 10,5 mm et 12,5 mm. La symétrie des boucles haptiques combinée avec cette limitation de leur déformabilité élimine le risque de rotation indésirable de la lentille sous l'effet des forces qui s'exercent sur elle (contraction des structures intérieures de l'oeil, mouvements de l'oeil, frottements exercés sur l'oeil).

L'invention est encore remarquable par les caractéristiques ci-après :
- les boucles haptiques sont reliées à la périphérie de la partie optique par des pédoncules ;
- le segment supérieur est formé par au moins deux tronçons reliés entre eux par un point de fléchissement ;
- en condition de compression maximale des boucles haptiques le diamètre hors tout de la lentille est compris entre 10,5 mm et 12,5 mm ;
- les points de fléchissement entre les segments des boucles haptiques et entre les dites boucles et la partie optique sont réalisés par amincissement de la section des dits segments ;
- la lentille comporte deux groupes de deux boucles haptiques symétriquement réparties à la périphérie de la partie optique ;
- les pédoncules comportent des saillies ou des creusures faisant fonction de détrompeur ;
- le bord de la face arrière de la partie optique comporte des saillies ou plots d'écartement.

L'invention sera mieux comprise à l'aide de la description qui va suivre d'un exemple de réalisation non limitatif en référence aux dessins annexés dans lesquels :
- la Figure 1 est un schéma de principe illustrant la géométrie et la cinématique particulières des haptiques conformes à l'invention pour un mode de réalisation dans lequel une boucle haptique est constituée par quatre segments reliés entre eux par des points de fléchissement : deux segments supérieurs et deux segments inférieurs ;
- La Figure 2 est une vue en plan de la face postérieure d'un implant de sulcus selon l'invention, dans un exemple de réalisation dans lequel une boucle haptique est constituée par quatre segments reliés entre eux par des points de fléchissement : deux segments supérieurs et deux segments inférieurs ;
- La Figure 3 est une vue en perspective de la face postérieure de l'implant de sulcus montré à la Figure 2 ;
- La Figure 4 est une vue en perspective de la face antérieure de l'implant de sulcus montré à la Figure 2 ;
- La Figure 5 est une vue en coupe selon le plan vertical médian de l'implant de sulcus montré à la Figure 2 ;
- La Figure 6 est une en perspective de la combinaison d'un implant de sulcus montré à la Figure 2 avec un implant de sac capsulaire ;
- La Figure 7 est une vue analogue à la Figure 2 d'un mode particulier de réalisation dans lequel la partie supérieure des boucles haptiques de l'implant de sulcus présente un contour crénelé ;
- La Figure 8 est un schéma de principe illustrant la géométrie et la cinématique particulières des haptiques conformes à l'invention pour un mode de réalisation dans lequel une boucle haptique est constituée par trois segments reliés entre eux par des points de fléchissement : un segment supérieur arqué et deux segments inférieurs ;
- La Figure 9 est une vue en plan de la face postérieure d'un implant de sulcus selon l'invention selon la Figure 8.

Dans les différentes figures, les éléments identiques ou fonctionnellement analogues sont indiqués par les mêmes références.

Sur les Figures la référence 1 désigne la partie optique et la référence 2 la partie haptique.

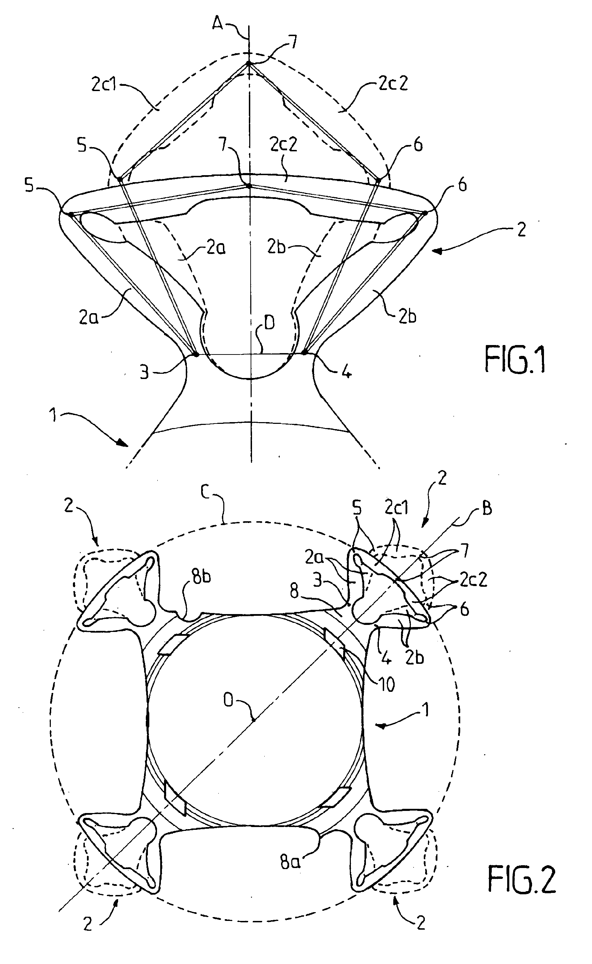

La Figure 1 illustre schématiquement une boucle haptique conforme à l'invention au repos (traits pointillés) et en état de déformation maximale (traits pleins) avec représentation des portions de droite illustrant ces différentes positions. On y voit que la boucle 2 est formée par deux segments inférieurs 2a, 2b qui se raccordent à une extrémité par deux points de fléchissement inférieurs 3, 4 à la périphérie de la partie optique 1, partiellement représentée sur le schéma, et, à l'autre extrémité, par deux points de fléchissement supérieurs 5,6 à un segment supérieur constitué par deux parties 2c1, 2c2 reliées entre elles par un point de fléchissement 7. Les points de fléchissement inférieurs 3, 4 sont écartés d'une distance D telle qu'ils convergent en direction de la partie optique et divergent en direction du segment supérieur 2c1, 2c2 par rapport à l'axe vertical médian A passant par le pôle d'une boucle haptique 2 et par l'axe optique de la lentille. La somme des longueurs des segments inférieurs 2a, 2b et de la distance D est supérieure à la longueur du segment supérieur 2c1 2c2. Sous l'effet de forces de compression s'exerçant sur le pôle de la boucle 2, sensiblement au point de fléchissement 7, en direction de la partie optique, le pivotement des segments inférieurs 2a, 2b produit l'abaissement des points de fléchissement supérieurs 5, 6 et l'aplatissement du segment 2c1, 2c2. Par construction, la boucle haptique 2 ne peut pas continuer à se déformer au-delà de la position basse représentée sur la Figure 1.

Ainsi, on obtient une boucle haptique 2 qui peut se déformer élastiquement en direction de la partie optique sur une distance limitée, cette déformation étant bloquée lorsque l'écartement maximal des segments inférieurs est atteint.

En pratique, les dimensions des segments constituant la boucle haptique 2 et l'écartement D des points de raccordement des segments inférieurs 2a, 2b à la partie optique sont choisis de telle sorte que le diamètre hors tout de la lentille à l'état de compression maximale de ses boucles haptiques 2 soit compris entre 10,5 mm et 12, 5 mm.

Sur la Figure 2 est représenté en plan une vue de la face postérieure d'un exemple de réalisation d'un implant de sulcus conforme au schéma de principe de la Figure 1.

L'implant de sulcus comporte quatre boucles haptiques 2 régulièrement réparties autour de la périphérie de la partie optique 1. Les boucles haptiques 2 étant identiques, les chiffres de référence n'ont été portés que sur l'une d'entre elles afin de ne pas alourdir le dessin. Les boucles haptiques 2 sont représentées en traits pleins (position comprimée) et en traits tiretés (position de repos). Chaque boucle 2 se raccorde à ladite partie optique par un pédoncule plat 8, de forme générale triangulaire, d'une épaisseur d'environ 0,2 mm. Les boucles haptiques 2 forment deux groupes diamétralement opposés par rapport à un axe transversal médian B passant par l'axe optique O. Le pédoncule 8 de l'une des deux boucles haptiques de chaque groupe présente une saillie latérale 8a, ou une creusure 8b faisant fonction de détrompeur.

Le cercle concentrique C symbolise la position de compression maximale des boucles haptiques 2. En pratique, le diamètre du cercle C sera compris entre 10,5mm et 12,5 mm.

Les segments inférieurs 2a, 2b ont de préférence une longueur de l'ordre de 1,6 mm et les segments supérieurs 2c1, 2c2 une longueur de l'ordre de 1,4 mm. Les points de fléchissement 3, 4, 5, 6 et 7 sont de préférence obtenus par diminution de la section du matériau constitutif de chaque boucle haptique 2.

Sous l'effet des forces de compression exercées sur les boucles haptiques 2, celles-ci se déforment selon un mouvement progressif d'aplatissement des segments supérieurs 2c1, 2c2 et d'écartement des segments inférieurs 2a, 2b autour de points de fléchissement 3, 4, 5, 6 et 7. Le rapport de dimensions des segments inférieurs par rapport aux segments supérieurs, leur écartement et leur convergence en direction de l'axe optique O assurent un blocage du mouvement de déformation au-delà d'un point de rebroussement dans lequel les segments supérieurs 2c, 2d sont sensiblement en alignement l'un avec l'autre.

De préférence, les lentilles sont réalisées dans un matériau acrylique.

Les pédoncules 8 plats et minces ont pour effet d'empêcher un risque de capture irienne par les boucles haptiques.

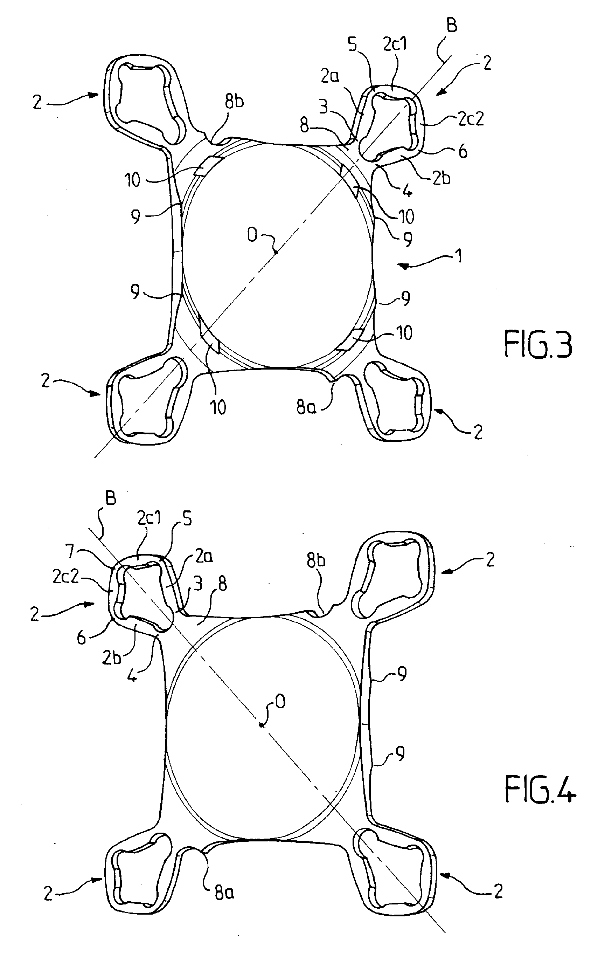

La lentille représentée sur les figures 2, 3 et 4 est une lentille additionnelle destinée à être positionnée dans le sulcus ciliaire en avant d'une lentille de sac capsulaire. Sa partie optique 1 est, de manière connue en soi, de forme concavo-convexe afin de s'adapter à la face avant de la lentille principale présente dans le sac capsulaire. Pour conserver entre les faces adjacentes des parties optiques de la lentille principale et de la lentille additionnelle un écartement permettant la circulation de l'humeur aqueuse, le bord arrière de la partie optique de la lentille de sulcus présente quatre saillies ou plots 9 disposés régulièrement sur le pourtour de ladite partie optique. Dans ce même but, à la périphérie de la face postérieure de la partie optique1, sensiblement centrés sur les diamètres passant par les pôles des boucles haptiques 2 sont en outre disposés des évidements 10.

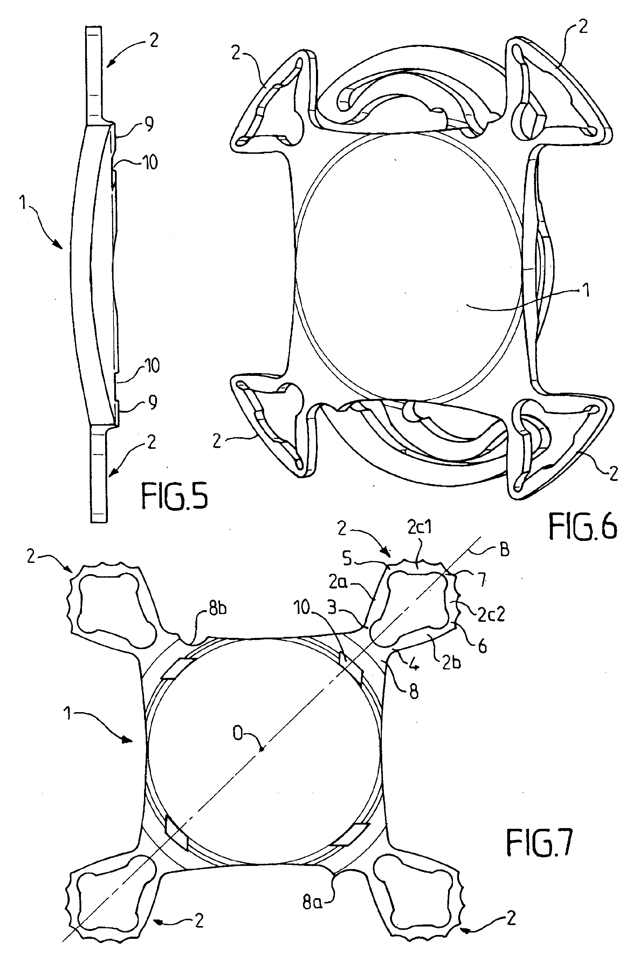

La Figure 7 illustre une variante de réalisation d'une lentille selon les Figures 2 à 6 dans laquelle la partie supérieure des boucles haptiques 2 de l'implant de sulcus présente un contour crénelé. Cette disposition procure une meilleure prise de cette partie des boucles haptiques avec la périphérie interne du sulcus ciliaire et par tant une meilleure tenue de la lentille.

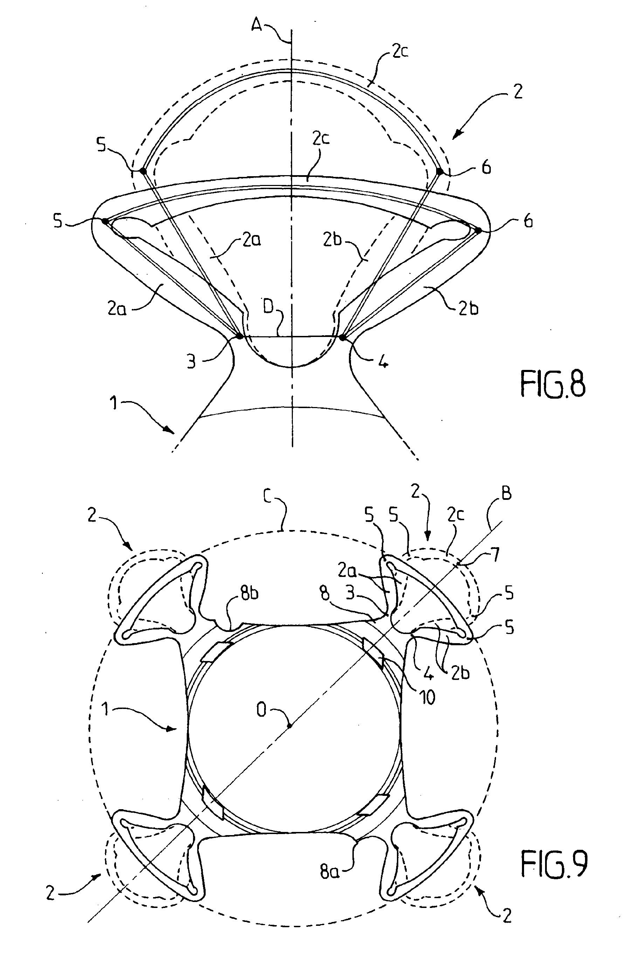

La Figure 8 représente schématiquement un deuxième mode de réalisation d' une boucle haptique conforme à l'invention au repos (traits pointillés) et en état de déformation maximale (traits pleins) avec représentation des portions de droite illustrant ces différentes positions. A la différence du schéma de la Figure 1, dans cet exemple de réalisation, la partie supérieure de la boucle haptique est constitué par un seul segment arqué 2c, au lieu de deux segments reliés par un point de fléchissement. Toutes les autres caractéristiques de la lentille sont semblables et sont repérées par les mêmes chiffres de référence. Le segment supérieur 2c est arqué de sorte que, sous l'effet de forces de compression en direction de la partie optique, le pivotement des segments inférieurs 2a, 2b produit l'abaissement des points de fléchissement supérieurs 5, 6 et l'aplatissement du segment 2c. Comme dans le mode de réalisation décrit en relation avec les Figures 1 à 7, la boucle haptique 2 ne peut pas continuer à se déformer au-delà de la position basse représentée sur la Figure 8.

La Figure 9 représente en vue en plan la face postérieure d'une lentille conforme au schéma de la Figure 8. Comme dans le mode de réalisation précédent, il s'agit d'une lentille additionnelle destinée à être positionnée dans le sulcus ciliaire en avant d'une lentille de sac capsulaire. Elle peut de même être réalisée avec un contour crénelé de la face de contact de la partie supérieure de ses boucles haptiques 2 avec la périphérie interne du sulcus ciliaire.

L'invention ici décrite dans un exemple d'application à un implant additionnel de sulcus, n'est pas limitée à cette forme de réalisation. Elle est également applicable à toute lentille intraoculaire destinée à être implantée dans le sillon irido-cornéen de la chambre antérieure. De même, dans les exemples de réalisation représentés sur les Figures, les lentilles de sulcus comportent quatre boucles haptiques régulièrement réparties à la circonférence de la partie optique. En variante, une lentille conforme à l'invention peut comporter seulement deux ou trois boucles haptiques régulièrement réparties à la circonférence de la partie optique.

## Revendications

1. Lentille intraoculaire destinée à être implantée dans le sulcus ciliaire de l'oeil et qui comporte une partie optique (1) à la périphérie de laquelle sont disposées au moins deux anses haptiques (2) en forme de boucles fermées disposées symétriquement de part et d'autre de la partie optique, chaque boucle (2) étant formée par au moins trois segments, un segment supérieur arqué (2c) et deux segments inférieurs (2a, 2b) assurant la liaison entre le segment supérieur (2c) et la partie optique (1), lesdits segments inférieurs étant convergents en direction de la partie optique, **caractérisée en ce que** les segments de chaque boucle (2) sont raccordés entre eux par des points de fléchissement (5, 6), **en ce que** les deux segments inférieurs (2a, 2b) se raccordent à la partie optique (1) de la lentille par des points de fléchissement (3, 4), et **en ce que** la longueur du segment supérieur (2c) est inférieure à la somme des longueurs des segments inférieurs (2a, 2b) et de leur écartement (D) au niveau de leur raccordement à la partie optique.

2. Lentille intraoculaire selon la revendication 1, **caractérisée en ce que** le segment supérieur (2a) est formé par au moins deux tronçons (2c1, 2c2) reliés entre eux par un point de fléchissement (7).

3. Lentille intraoculaire selon la revendication 1 ou 2, **caractérisée en ce que** en condition de compression maximale des boucles haptiques son diamètre hors tout est compris entre 10,5 mm et 12,5 mm.

4. Lentille intraoculaire selon l'une quelconque des revendications 1 à 3, **caractérisée en ce que** les points de fléchissement (5, 6) entre les segments (2a, 2b, 2c) des boucles haptiques (2) et (3, 4) entre les dites boucles et la partie optique (1) sont réalisés par amincissement de la section des dits segments.

5. Lentille intraoculaire selon l'une quelconque des revendications 1 à 4 **caractérisée en ce qu'**elle comporte deux groupes de deux boucles haptiques (2) symétriquement réparties à la périphérie de la partie optique (1).

6. Lentille intraoculaire selon l'une quelconque des revendications là 5, **caractérisée en ce que** les boucles haptiques (2) sont reliées à la périphérie de la partie optique (1) par des pédoncules (8).

7. Lentille intraoculaire selon la revendication 6, **caractérisée en ce que** les pédoncules comportent des saillies (8a) ou des creusures (8b) faisant fonction de détrompeur.

8. Lentille intraoculaire selon l'une quelconque des revendications 1 à 7, **caractérisée en ce que** le bord de la face arrière de la partie optique (1) comporte des saillies ou plots d'écartement (9).

9. Lentille intraoculaire selon l'une quelconque des revendications 1 à 8, **caractérisée en ce que** le bord de la face arrière de la partie optique (1) comporte des évidements (10) sensiblement centrés sur les diamètres passant par les pôles des boucles haptiques (2).

10. Lentille intraoculaire selon l'une quelconque des revendications 1 à 9, **caractérisée en ce que** la partie supérieure des boucles haptiques (2) présente un contour crénelé.

## Patentansprüche

1. Intraokularlinse, die zum Implantieren in den Sulcus ciliaris des Auges bestimmt ist und die einen optischen Teil (1) aufweist, an dessen Umfang mindestens zwei haptische Henkel (2) in Form geschlossener Schleifen angeordnet sind, die auf der einen und der anderen Seite des optischen Teils symmetrisch angeordnet sind, wobei jede Schleife (2) von mindestens drei Segmenten, einem oberen gebogenen Segment (2c) und zwei unteren Segmenten (2a, 2b), die die Verbindung zwischen dem oberen Segment (2c) und dem optischen Teil (1) sichern, gebildet ist, wobei die unteren Segmente in Richtung des optischen Teils konvergierend sind, **dadurch gekennzeichnet, dass** die Segmente jeder Schleife (2) untereinander durch Biegepunkte (5, 6) verbunden sind, dass sich die zwei unteren Segmentes (2a, 2b) mit dem optischen Teil (1) der Linse durch Biegepunkte (3, 4) verbinden und dass die Länge des oberen Segments (2c) kleiner als die Summe der Längen der unteren Segmente (2a, 2b) und ihres Abstands (D) auf Ebene ihrer Verbindung mit dem optischen Teil ist.

2. Intraokularlinse nach Anspruch 1, **dadurch gekennzeichnet, dass** das obere Segment (2a) von mindestens zwei Abschnitten (2c1, 2c2) gebildet ist, die untereinander durch einen Biegepunkt (7) verbunden sind.

3. Intraokularlinse nach Anspruch 1 oder 2, **dadurch gekennzeichnet, dass** ihr Durchmesser über alles bei maximaler Kompression der haptischen Schleifen zwischen 10,5 mm und 12,5 mm inklusive ist.

4. Intraokularlinse nach einem der Ansprüche 1 bis 3, **dadurch gekennzeichnet, dass** die Biegepunkte (5, 6) zwischen den Segmenten (2a, 2b, 2c) der haptischen Schleifen (2) und (3, 4) zwischen den Schleifen und dem optischen Teil (1) durch Verschlankung des Querschnitts der Segmente hergestellt sind.

5. Intraokularlinse nach einem der Ansprüche 1 bis 4, **dadurch gekennzeichnet, dass** sie zwei Gruppen aus zwei haptischen Schleifen (2) aufweist, die auf dem Umfang des optischen Teils (1) symmetrisch verteilt sind.

6. Intraokularlinse nach einem der Ansprüche 1 bis 5, **dadurch gekennzeichnet, dass** die haptischen Schleifen (2) mit dem Umfang des optischen Teils (1) durch Stiele (8) verbunden sind.

7. Intraokularlinse nach Anspruch 6, **dadurch gekennzeichnet, dass** die Stiele Vorsprünge (8a) oder Aushöhlungen (8b) aufweisen, die eine Unverwechselbarkeitsfunktion erfüllen.

8. Intraokularlinse nach einem der Ansprüche 1 bis 7, **dadurch gekennzeichnet, dass** der Rand der Rückseite des optischen Teils (1) Beabstandungsvorsprünge oder -kontakte (9) aufweist.

9. Intraokularlinse nach einem der Ansprüche 1 bis 8, **dadurch gekennzeichnet, dass** der Rand der Rückseite des optischen Teils (1) Aussparungen (10) aufweist, die etwa auf den durch die Pole der haptischen Schleifen (2) laufenden Durchmessern zentriert sind.

10. Intraokularlinse nach einem der Ansprüche 1 bis 9, **dadurch gekennzeichnet, dass** der obere Teil der haptischen Schleifen (2) eine gezackte Kontur aufweist.

## Claims

1. An intraocular lens designed to be implanted in the ciliary sulcus of the eye and which comprises an optical part (1) on the periphery of which at least two haptic loops (2) are positioned in the form of closed loops positioned symmetrically on either side of the optical part, each loop (2) being formed by at least three segments, a bowed upper segment (2c) and two lower segments (2a, 2b) providing the connection between the upper segment (2c) and the optical part (1), said lower segments converging toward the optical part, **characterized in that** the segments of each loop (2) are connected to each other by sag points (5, 6), **in that** the two lower segments (2a, 2b) are connected to the optical part (1) of the lens by sag points (3, 4), and **in that** the length of the upper segment (2c) is smaller than the sum of the lengths of the lower segments (2a, 2b) and their separation (D) at their connection to the optical part.

2. The intraocular lens according to claim 1, **characterized in that** the upper segment (2a) is formed by at least two sections (2c1, 2c2) connected to each other by a sag point (7).

3. The intraocular lens according to claim 1 or 2, **characterized in that** under maximum compression conditions of the haptic loops, its overall diameter is comprised between 10.5 mm and 12.5 mm.

4. The intraocular lens according to any one of claims 1 to 3, **characterized in that** the sag points (5, 6) between the segments (2a, 2b, 2c) of the haptic loops (2) and (3, 4) between said loops and the optical part (1) are made by thinning the section of said segments.

5. The intraocular lens according to any one of claims 1 to 4, **characterized in that** it comprises two groups of haptic loops (2) distributed symmetrically on the periphery of the optical part (1).

6. The intraocular lens according to any one of claims 1 to 5, **characterized in that** the haptic loops (2) are connected to the periphery of the optical part (1) by peduncles (8).

7. The intraocular lens according to claim 6, **characterized in that** the peduncles comprise protrusions (8a) or hollows (8b) serving as mistake proofing means.

8. The intraocular lens according to any one of claims 1 to 7, **characterized in that** the edge of the rear face of the optical part (1) comprises protrusions or separating studs (9).

9. The intraocular lens according to any one of claims 1 to 8, **characterized in that** the edge of the rear face of the optical part (1) comprises recesses (10) substantially centered on the diameters passing through the poles of the haptic loops (2).

10. The intraocular lens according to any one of claims 1 to 9, **characterized in that** the upper part of the haptic loops (2) has a crenellated contour.
